# EUROPEAN PATENT APPLICATION

(11) **EP 4 699 663 A2**
(43) Date of publication of application: **25.02.2026**
(21) Application number: 25219737.1
(22) Date of filing: 29.01.2019
(51) Int. Cl.: A61P 37/06

(54) **PARENTERAL FORMULATION MATERIALS AND METHODS FOR 40-O-CYCLIC HYDROCARBON ESTERS AND RELATED STRUCTURES**

(30) Priority: 23.02.2018 US 201862634212 P
(62) Divisional of application: 19702558.8
(71) Applicant: BIOTRONIK AG, 8180 Bülach (CH)
(72) Inventor: BETTS, Ronald E., La Jolla, 92037 (US)
(74) Representative: WSL Patentanwälte Partnerschaft mbB

(57) **Abstract**

This application relates generally to the field of drug treatment paradigms based on specifically formulated compounds for use in targeted therapy or disease prevention. Specifically, this technology provides for compositions and methods for treating, stabilizing, preventing or delaying disease conditions through administration of highly lipophilic compositions with a globular serum protein in combination with other pharmaceutical compositions.

## Description

### FIELD OF THE INVENTION

This application relates generally to the field of drug treatment paradigms based on specifically formulated compounds for use in targeted therapy or disease prevention. Specifically, this technology provides for compositions and methods for treating, stabilizing, preventing or delaying disease conditions through administration of highly lipophilic compositions with a globular serum protein in combination with other pharmaceutical compositions.

### BACKGROUND OF THE INVENTION

Pharmaceutical formulations having active pharmaceutical ingredients (APIs) with low water solubility characteristics has been an issue in the drug development industry for years. Furthermore, developing such problematic formulations into soluble parenteral dosage forms has been extremely challenging in view of the many considerations that must be taken into account, including ingredient solubility (logP), stability, toxicity, desired final concentration, bioavailability, manufacturing cost and shelf life limitations, to name just a few.

Numerous excipients have been developed to aid aqueous API solubility issues, including water soluble organic solvents, surfactants, fats and oils, as well as other materials. Many excipients are used together in combination with the API and optimum ratios, which is largely borne out of many trial and error experiments.

Compositions and formulations involving rapamycin (sirolimus) and related derivatives is no exception to the aforementioned problems with respect to solubility.

US Patent Nos. 5,616,588 and 5,516,770 describe the problems of rapamycin with respect to solubility. Specifically, rapamycin was shown to be insoluble in water and only slightly soluble in commonly used solubilizers, including propylene glycol, glycerin and polyethylene glycol.

Some disclosures in the prior art purport to resolve the problems described previously with combining insoluble compounds, such as rapamycin, with nanoparticles. US Patent Publication No. US 2015/0050356 (8,911,786) teaches methods for the treatment of cancer using nanoparticles that comprise rapamycin or a derivative thereof. Similarly, nanoparticles formulated using a diblock copolymer, polyethylene glycol-poly-l-lactic acid (mPEG-PLA), monovalent metal salt of a biodegradable polyester (D,L-PLACOONa), and calcium chloride has been reported (Ha et al., Int. J. Nanomed, 2012:7, 2197-2208). However, it is a requirement of these teachings that nanoparticles comprising rapamycin be used in the treatment paradigm, versus having a formulation without nanoparticles.

Rapamycin is an mTOR inhibitor that has a history of being included in parenteral formulations. WO 2004/011000 teaches parenteral formulations containing rapamycin 42-ester with 3-hydroxy-2-(hydroxymethyl)-2-methylpropionic acid (known as CCI-779). However, CCI-779, as a parenteral formulation, has significant problems to overcome in view of the poor aqueous solubility problems described supra. These problems were meant to be solved by solubilizing CCI-779 with a cosolvant, further accompanied by an antioxidant or chelating agent in solution, as well as a parenterally acceptable surfactant. This overly complicated means of solving the solubility problems results in the addition of far too many elements into the parenteral formulation.

There is a need in the state of the art to improve the poor solubility aspects to rapamycin-based parenteral formulations without the need for additions of multiple cosolvents and surfactants into the final formulation.

### SUMMARY OF THE INVENTION

The present invention provides for a drug formulation comprising a first, a second and a third component, the first component comprising at least one of a macrocyclic triene immunosuppressive compound selected from the group comprising or consisting of rapamycin (sirolimus), everolimus, zotarolimus, biolimus, novolimus, myolimus, temsirolimus, derivatives related thereto and a compound having the structure: 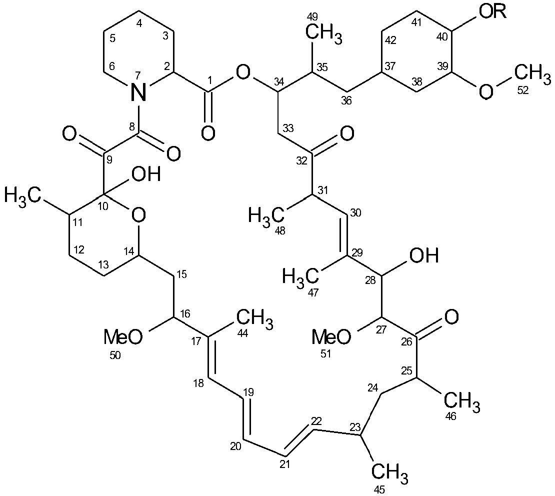 where R is C(O)-(CH₂)ₙ-X, n is 0, 1 or 2, X is a cyclic hydrocarbon having 3-9 carbons, optionally containing one or more unsaturated bonds, the second component comprising at least one water soluble solubilizer, wherein the first component is solubilized in the second component, and the third component comprising a water soluble polymer. In a further embodiment X is a cyclic hydrocarbon having 3-7 carbons. In one embodiment the first component comprises only one macrocyclic triene immunosuppressive compound as described above. Preferably, the at least one water soluble solubilizer is selected from the group comprising or consisting of ethyl alcohol (EtOH), propylene glycol, one or more polyoxyethylene soribitan esters, polyethylene glycol 200, 300, 400 or combinations thereof. In one embodiment the second component consists of only one member of the water soluble solubilizers as defined above. In another embodiment the second component comprises more than one member of the water soluble solubilizers as defined above and is composed of a mixture of water soluble solubilizers as defined above. In a preferred embodiment, the macrocyclic triene immunosuppressive compound has the structure: 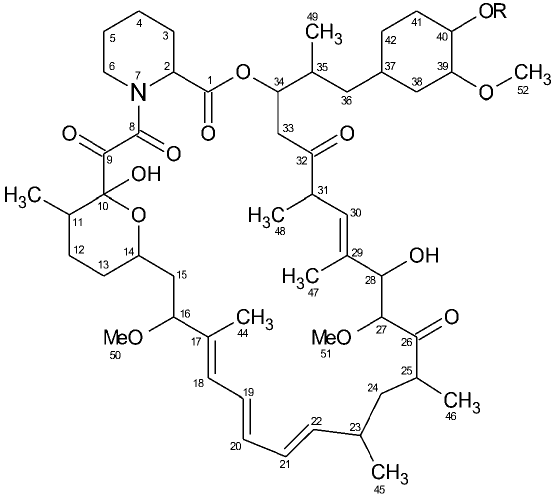 as defined above and R being C(O)-(CH₂)ₙ-X has one of the following structures:

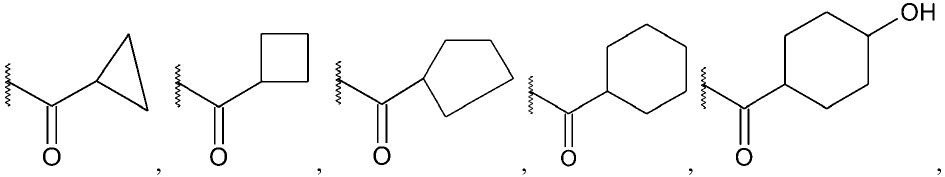

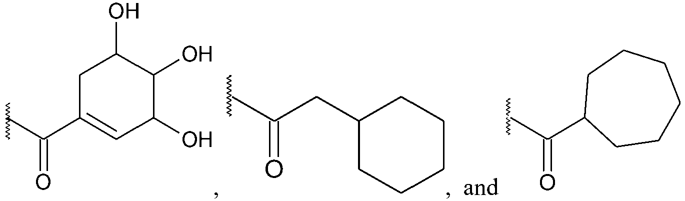

Further, the formulation comprises a third component containing a water soluble polymer and an aqueous solvent, wherein the first and second components are dispensed in a solution. In one embodiment the water soluble polymer is a protein having an approximate molecular weight of between 50 to 200 kD. In one embodiment the water soluble polymer is selected from water soluble human serum proteins or water soluble blood proteins preferably having an approximate molecular weight of between 50 to 200 kD. In one embodiment the water soluble polymer is a protein having an approximate molecular weight of between 65-70 kD, most preferably a globular serum protein having an approximate molecular weight of between 65-70 kD. In a further embodiment the water soluble material is selected from blood proteins such as globulins and/or fibrinogens having molecular weights up to approximately 160 kD, preferably of human origin. Also, in a preferred embodiment the third component comprises or consists of the water soluble polymer as suggested herein as an aqueous solution, preferably dissolved in physiological saline.

In one aspect, the present invention provides for a method of manufacturing a drug formulation as suggested herein for parenteral administration comprising: (a) providing a first component comprising at least one macrocyclic triene immunosuppressive compound as suggested herein and preferably having the structure: 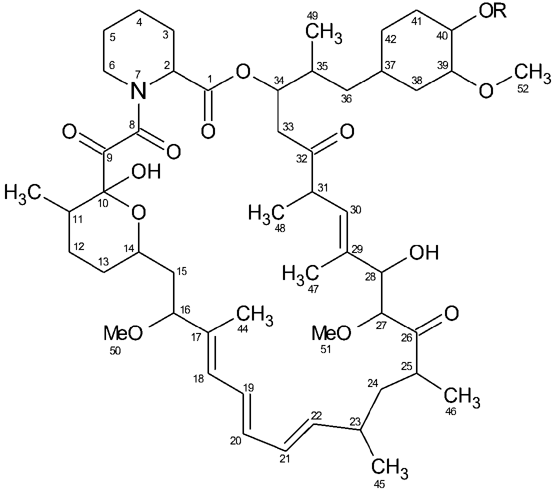 where R is C(O)-(CH₂)ₙ-X, n is 0, 1 or 2, X is a cyclic hydrocarbon having 3-9 carbons, optionally containing one or more unsaturated bonds; (b) solubilizing the component of (a) in a second component comprising an effective amount of a water soluble solubilizer; (c) dispensing the product of (b) in a third component comprising a water soluble polymer. In a further embodiment X is a cyclic hydrocarbon having 3-7 carbons. Preferably, the water soluble solubilizer is selected from the group comprising or consisting of ethyl alcohol (EtOH), propylene glycol, one or more polyoxyethylene soribitan esters, polyethylene glycol 200, 300, 400 or combinations thereof. Further preferably, the water soluble polymer is a human serum protein, and more preferably is human serum albumin.

### BRIEF DESCRIPTION OF THE FIGURES

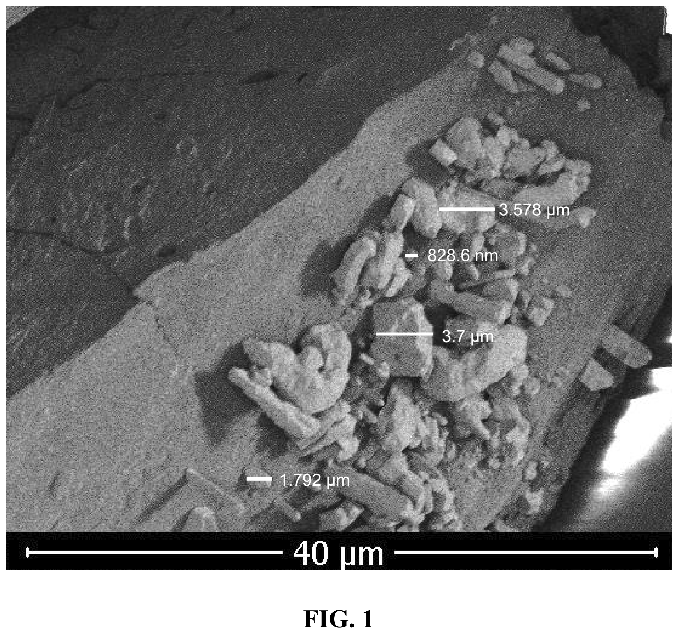

Figure 1 depicts a photomicrograph of a scanning electron microscopy study of an I.V. solution containing a formulation as suggested herein. An I.V. solution containing a formulation as suggested herein was allowed to dry producing a solid film. To allow additional examination, mechanical abrasion of the film was conducted resulting in the observed irregular material. No uniform nanoparticles from the solution are observed. The sizes of the irregular particles measure from the top to the buttom of the figure 3.578 µm, 828.6 nm, 3.700 µm and 1.792 µm. In contrast for known parenteral formulations spherical uniformly sized regular nanoparticles have to be formed such as those published as a photomicrograph of a scanning electron microscopy study of nanospheres (Gu. et al., ACS Nano, 2013:7(5), 4194-4201).

### DETAILED DESCRIPTION OF THE INVENTION

As used herein, the term "macrocyclic triene immunosuppressive compound" includes rapamycin (sirolimus), everolimus, zotarolimus, biolimus, novolimus, myolimus, temsirolimus and the rapamycin derivatives described in this disclosure.

The present invention provides for a solution to the solubility issues related to formulations comprising highly lipophilic compounds as the API with a pharmaceutical product. The state of the art in this field utilizes a variety of excipients to aid aqueous API solubility. A list of known excipients to accommodate such use appears below in Table 1.

**Table 1**

| Commercially Available, Solubilizing Excipients for Use in Oral and Injectable Formulations | | |
|---|---|---|
| **Water-soluble** | **Water-insoluble** | **Surfactants** |
| Dimethylacetamide (DMA) | Beeswax | Polyoxyl 35 castor oil |
| Dimethyl sulfoxide (DMSO) | Oleic acid | Polyoxyl 40 hydrogenated castor oil |
| Ethanol | Soy fatty acids | Polyoxyl 60 hydrogenated castor oil |
| Glycerin | Vitamin E | Polysorbate 20 (Tween 20) |
| N-methyl-2-pyrrolidone (NMP) | Corn oil mono-di-tridiglycerides | Polysorbate 80 (Tween 80) |
| PEG 300 | Medium chain diglycerides | *d*-α-tocopheryl polyethylene glycol |
| PEG 400 | Long chain triglycerides | Solutol HS-15 |
| Poloxamer 407 | Medium chain triglycerides | Sorbitan monooleate (Span 20) |
| Propylene glycol | | PEG 300 caprylic/capric glycerides |
| Hydroxypropyl-β-cyclodextrin | | PEG 400 caprylic/capric glycerides |
| Sulfobutylether-β-cyclodextrin | | PEG 300 oleic glycerides |
| Phospholipids | | PEG 300 linoleic glycerides |
| | | Polyoxyl 8 stearate |
| | | Polyoxyl 40 stearate |
| | | Peppermint oil |

In the present invention, the stability of the drug formulation depends on the combination of a first component comprising a macrocyclic triene immunosuppressive compound together with a second component being or comprising a water soluble solubilizer. The macrocyclic triene immunosuppressive compound may be selected from the group consisting of rapamycin (sirolimus), everolimus, zotarolimus, biolimus, novolimus, myolimus, temsirolimus and derivatives related thereto. Preferably, the macrocyclic triene immunosuppressive compound of the present invention is a rapamycin 40-ester analog having the following structure: 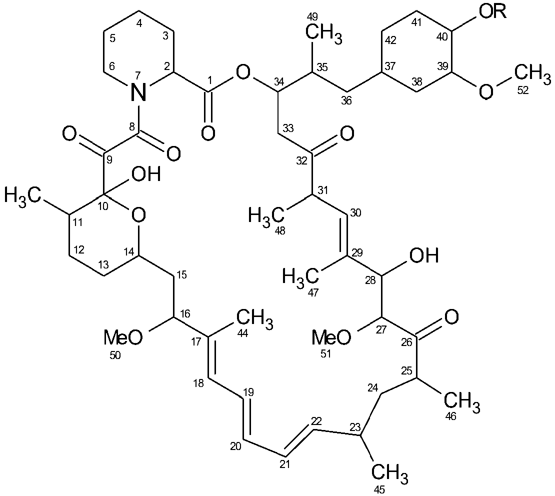 where R is C(O)-(CH₂)ₙ-X, n is 0, 1 or 2, X is a cyclic hydrocarbon having 3-9 carbons and optionally contains one or more unsaturated bonds. In a further embodiment X is a cyclic hydrocarbon having 3-7 carbons. In a most preferred embodiment, C(O)-(CH₂)ₙ-X has one of the following structures: 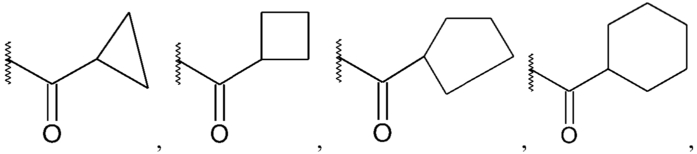 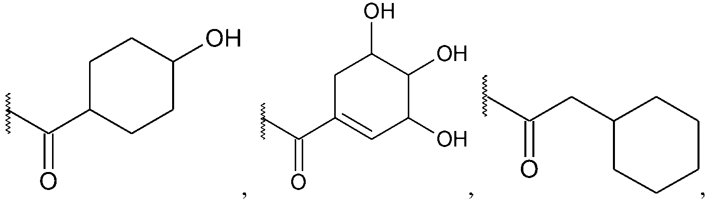 and

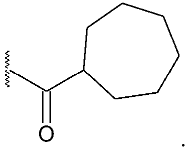

In a further embodiment, the first component of the formulation as suggested herein may comprise at least one member of the group consisting of rapamycin (sirolimus), everolimus, zotarolimus, biolimus, novolimus, myolimus, temsirolimus, and may further comprise one component having the following structure: 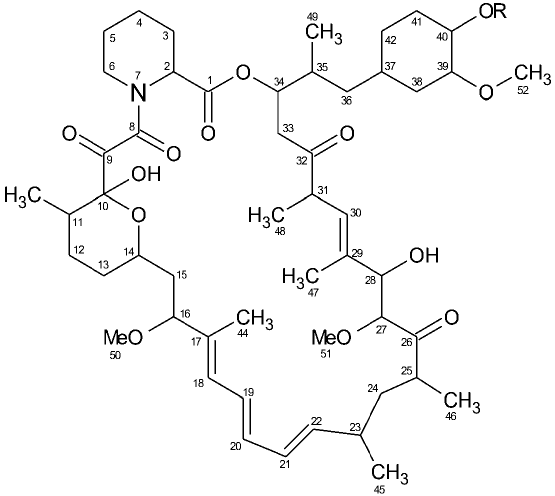 where R is C(O)-(CH₂)ₙ-X, n is 0, 1 or 2, X is a cyclic hydrocarbon having 3-9 carbons and optionally contains one or more unsaturated bonds, as described herein. Thereby, the first component may comprise or consist of a mixture of macrocyclic triene immunosuppressive compounds as described herein.

The second component may be a water soluble solubilizer. In a preferred embodiment, the water soluble solubilizer is selected from the group comprising or consisting of ethyl alcohol (EtOH), propylene glycol, one or more polyoxyethylene soribitan esters, polyethylene glycol 200, 300, 400 or combinations thereof. In one embodiment the second component consists of only one member the group as defined above, and preferably comprises or consists of ethanol. In another embodiment the second component comprises more than one member of the group as defined above. In one embodiment, the second component comprises two, three four or five members of the group defined above. In a preferred embodiment, the second component comprises two members of the group as defined above, and more preferably comprises or consists of propylene glycol and a polysorbate, preferably polysorbate 80, preferably in a 50/50 wt-% mixture.

Preferably, the formulation is further comprised of a third component, into which the first and second components are dispensed, wherein the third component comprises a water soluble polymer. In one embodiment the water soluble polymer is a protein having an approximate molecular weight of between 50 to 200 kD. In one embodiment the water soluble polymer is selected from water soluble human serum proteins or water soluble blood proteins preferably having an approximate molecular weight of between 50 to 200 kD. In one embodiment the water soluble polymer is a protein having an approximate molecular weight of between 65-70 kD, most preferably a globular serum protein having an approximate molecular weight of between 65-70 kD. In a further embodiment the water soluble material is selected from blood proteins such as globulins and/or fibrinogens having molecular weights up to approximately 160 kD, preferably of human origin. However, in a most preferred embodiment, the water soluble polymer is a human serum protein having at least 90% identity to the following sequence:











 (SEQ ID NO: 1)

In a most preferred embodiment the water soluble polymer is human serum albumin.

The water soluble polymer as part of the third component is preferably provided in the formulation as an aqueous solution. In one embodiment the solution is based on water, preferably sterilized water. In a preferred embodiment the third component is provided for the formulation as suggested herein as a solution of the water soluble polymer in physiological saline. Physiological saline is known to the skilled person as a 0.9% (wt/vol) solution of NaCl in water, usually displaying a pH of 4.5 to 7.0.

In one embodiment the formulation is suggested herein comprises or consists of 0.01 to 5 wt-% of the first component, 5 to 20 wt-% of the second component and 70 to 95 wt-% of the third component. The third component may preferably be provided as a 5 to 40% (wt/vol) aqueous solution of the water soluble polymer, preferably in physiological saline. Also, if no further components are added to the formulation the above figures add up to 100 wt-%.

Additionally, the present invention provides for a method of manufacturing a drug formulation as suggested herein for parenteral administration comprising: (a) providing a first component comprising at least one of a macrocyclic triene immunosuppressive compound as suggested herein and preferably having the structure: 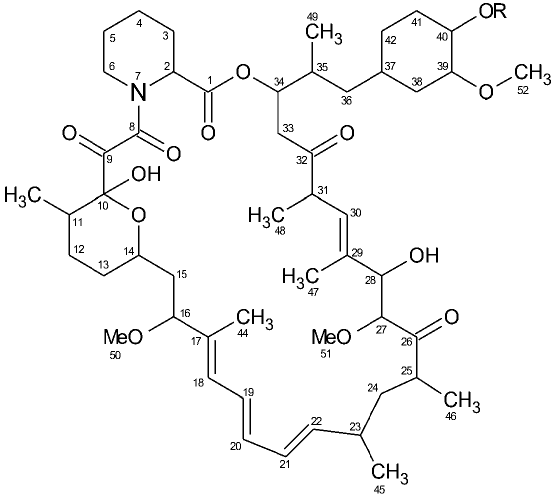 where R is C(O)-(CH₂)ₙ-X, n is 0, 1 or 2, X is a cyclic hydrocarbon having 3-9 carbons, optionally containing one or more unsaturated bonds; (b) solubilizing the component of (a) in a second component comprising an effective amount of a water soluble solubilizer; and (c) dispensing the product of (b) in a third component comprising a water soluble polymer.

To advantage by the method as described above formulations for parenteral administration can be provided which do not contain nanoparticles, in particular spherical uniformly sized regular nanoparticles of the macrocyclic triene immunosuppressive compound. Usually parenteral formulations comprise nanoparticles containing the active agent and a polymer as a carrier. Such nanoparticle formation is not required for the formulation and the method as suggested herein. The formulation and the method do not comprise an ingredient (formulation) or a step of forming (method) nanoparticles, in in particular spherical uniformly sized regular nanoparticles of the macrocyclic triene immunosuppressive compound and a polymer or any other carrier. Such forming of nanoparticles for injectable solutions is laborious and costly. Saving the formation of nanoparticles renders the method as presented herein cost- and labor efficient.

In a preferred embodiment of the formulation as suggested herein, the water soluble solubilizer is selected from the group comprising or consisting of ethyl alcohol (EtOH), propylene glycol, one or more polyoxyethylene soribitan esters, polyethylene glycol 200, 300, 400 or combinations thereof and the water soluble polymer is a human serum protein.

A further aspect of the invention as described herein is directed to an injectable aqueous solution comprising the formulation as suggested herein for use in parenteral administration to an individual in need thereof.

A further aspect of the invention as described herein is directed to a kit containing the first, the second and the third components as suggested herein in pre-weighed and/or premixed combinations thereof and in sterile container(s) to allow ready parenteral administration.

### Examples

### Example Formulations:

The macrocyclic triene immunosuppressive compound of the present invention has more than one embodiment and may be described as comprising at least one of the following species from Table 2:

**Table 2**

| Description of CRC-015 species | | |
|---|---|---|
| Main structure | R is C(O)-(CH2)n-X having one of the following structures | Species |
| 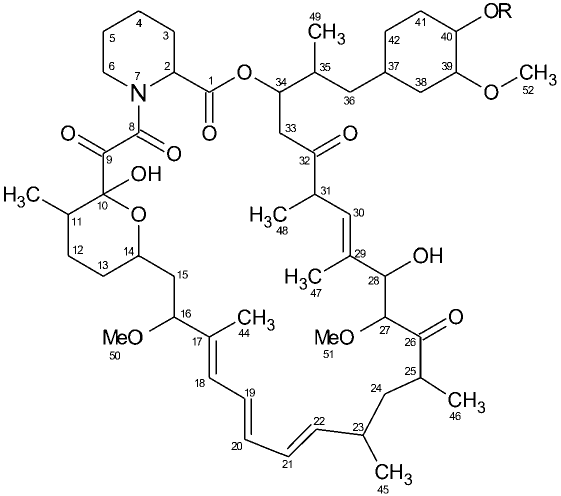 | 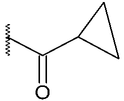 | CRC-015a |
| | 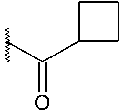 | CRC-015b |
| | 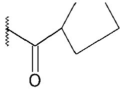 | CRC-015c |
| | 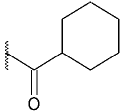 | CRC-015d |
| | 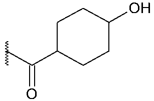 | CRC-015e |
| | 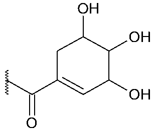 | CRC-015f |
| | 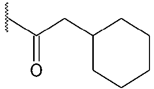 | CRC-015g |
| | 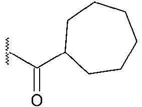 | CRC-015h |

CRC-015 is a term meant to encompass a genus and used to refer to each of the following species from Table 1: CRC-015a, CRC-015b, CRC-015c, CRC-015d, CRC-015e, CRC-015f, CRC-015g, and CRC-015h.

### I. Formulation of CRC-015:

The target compound CRC-015 is formulated in a particular manner, together with the water soluble solubilizer and the human serum protein. This formulation avoids the requirement of nanoparticles. The resulting formulation provides a simple parenteral dosage form that provides superior PK results when compared to previous studies examining rapamycin.

For this exemplary formulation, CRC-015 is dissolved in EtOH and further prepared as follows: 25mg/ml CRC-015/EtOH solution is directly dispensed into a 20% solution containing SEQ ID NO:1 (wt/vol) in physiological saline followed by brief stirring to prepare the dosing solution.

The below examples utilized a final formulated drug concentration of 2.67 mg CRC-015/ml of SEQ ID NO:1. It is understood the concentration may be optimized according to desired dosing schemes, routes of administration, etc.

### II. Pharmacokinetic Studies using CRC-015 Formulation

The PK studies were conducted around the formulation from Example I. Specifically, Sprague-Dawley rats were dosed intravenously at 15 mg/kg, with blood samples being collected prior to dosing, in order to establish baseline, then post dosing at set intervals up to 24 hours. Drug bioanalytical measurements were conducted by LCMS.

Results (AUC_{inf}) reported in the prior art are from blood plasma, whereas the present results are from both plasma and whole blood. This was done to allow for direct comparison to the previous studies. Drug area under the curve versus time ((AUC_{inf}), total drug dose exposure) was calculated accordingly.

The results for CRC-015 with SEQ ID NO:1 and without nanoparticles is described in Table 3.

**Table 3**

| PK Results from CRC-015 Dosing Studies | | |
|---|---|---|
| **Test Study** | **AUC_{inf} (hr*ng/ml)** | **AUC_{inf} / (mg/kg)** |
| CRC-015 Plasma [15 mg/kg], n=2 | 19824 ± 1888 | 1322 |
| CRC-015 Whole Blood [15 mg/kg], n=2 | 21184 ± 1141 | 1412 |

### III. Comparison Studies with Nanoparticle Formulations

Sirolimus (rapamycin) was used and formulated in accordance with those steps described previously at Example I. Specifically, sirolimus was combined with SEQ ID NO:1 but without nanoparticles and tested against the studies shown in the prior art, namely, sirolimus formulated with SEQ ID NO:1 but with nanoparticles. The results are described in Table 4.

**Table 4**

| PK Results from Sirolimus Dosing Studies with/without Nanoparticles | | |
|---|---|---|
| **Test Study** | **AUC_{inf} (hr*ng/ml)** | **AUC_{inf} / (mg/kg)** |
| Sirolimus Plasma (Current) [15 mg/kg], n=2 | 5180 ± 130 | 345 |
| Sirolimus Whole Blood (Current) [15 mg/kg], n=2 | 5060 ± 541 | 337 |
| Sirolimus (Prior Art) [15 mg/kg], n=4 | 6017 ± 647 | 401 |

The 20% difference between the AUC_{inf} results between the current studies and those described in the prior art could be attributable to a nanoparticle effect, or due to the variability between laboratories. However, what is clear is the surprising finding that the CRC-015 results from Table 3 (without nanoparticles) resulted in a greater than three-fold AUC when compared to the use of sirolimus nanoparticles of the prior art.. This unexpected result is very significant and translates to drug dosing with CRC-015 at a higher AUC/unit dose when compared to sirolimus or to similar AUC with a smaller dose.

The parenteral formulation materials of this disclosure were further evaluated using additional alternative water soluble solubilizers.

An intravenous concentrate (I.V. concentrate) solution was prepared by mixing 5 g propylene glycol (USP, Sigma-Aldrich P4347) with 5 g polysorbate 80 (NF, Spectrum PU13). Next, 50 mg CRC-015 was weighed into a 2 mL volumetric flask and the 50/50 propylene glycol, polysorbate 80 solution was added to the flask mark. The drug was dissolved by repeated inversion of the flask to yield an I.V. concentrate of 25 mg/mL CRC-015.

I.V. injection solutions were prepared by weighing 1.8 g human serum albumin (HSA) (Sigma-Aldrich A9731) and layering onto the top surface of approximately 7.5 mL sterile 0.9% saline solution (Teknova S5812) contained in a 25 mL beaker until dissolved. This solution was quantitatively adjusted to a final volume of 9 mL with sterile saline to yield a 20% wt/vol HSA solution. The 20% HSA solution was filter sterilized using a 0.20 um sterile filter (Fisherbrand 09-719C) and stored at 3°C until used. For final preparation of I.V. drug injection solutions, I.V. concentrate was added to the saline-HSA using a sterile 100 uL glass syringe followed by vortexing to yield I.V. injection solutions of approximately 0.4-0.5 mg/mL. Examination of these I.V. injection solutions and solutions of various higher or lower drug concentrations by scanning electron microscopy determined that the solutions were void of any nanoparticulate materials. Pharmacokinetic studies of these materials conducted with rats in a manner as previously described yielded results as follows below.

| **Test Study** | **AUC_{inf} (hr*ng/ml)** | **AUC_{inf} / (mg/kg)** |
|---|---|---|
| A. CRC-015 Whole Blood [2.1 mg/kg], n=2 | 2323 ± 195 | 1106 |
| B. CRC-015 Whole Blood [2.1 mg/kg], n=2 | 2298 ± 74 | 1094 |
| C. CRC-015 Whole Blood [15 mg/kg], n=3 | 28,159 ± 1529 | 1877 |

As evidenced in the aforementioned Examples, coupled with the premise that rat PK studies are often extrapolated to human PK expectations, the findings from the CRC-015 formulation studies directly suggest the value of such a parenteral formulation with respect to human mTOR treatment utility. For instance, such unique formulations may improve many parameters in treatment paradigms, including patient response, dosing regimens, drug-drug interactions, toxicities and overall patient care and outcome. The ability to eliminate drug nanoparticles is of particular importance, as it will greatly simplify the drug manufacturing process by removing further complicated manufacturing steps to accommodate the nanoparticle integration, as well as obviate the need for specialized equipment and unique chemicals required for nanoparticle formulations. For example, the potential use of chloroform when preparing formulations involving nanoparticles can now be removed as a compound in the manufacturing process, which is advantageous in view of the known issues around chloroform's adverse impact on stability within this lipophilic class of compounds. Also reduced or removed is the use of various materials and synthetic polymers that may have various human toxicological considerations.

The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed. Thus, it should be understood that although the present invention has been specifically disclosed by preferred embodiments and optional features, modification and variation of the inventions herein disclosed can be resorted by those skilled in the art, and that such modifications and variations are considered to be within the scope of the inventions disclosed herein. The inventions have been described broadly and generically herein. Each of the narrower species and subgeneric groupings falling within the scope of the generic disclosure also form part of these inventions. This includes the generic description of each invention with a proviso or negative limitation removing any subject matter from the genus, regardless of whether or not the excised materials specifically resided therein.

In addition, where features or aspects of an invention are described in terms of the Markush group, those schooled in the art will recognize that the invention is also thereby described in terms of any individual member or subgroup of members of the Markush group. It is also to be understood that the above description is intended to be illustrative and not restrictive. Many embodiments will be apparent to those of ordinary skill in the art upon reviewing the above description. The scope of the invention should therefore, be determined not with reference to the above description, but should instead be determined with reference to the appended claims, along with the full scope of equivalents to which such claims are entitled. The disclosures of all articles and references, including patent publications, are incorporated herein by reference.

It will be apparent to those skilled in the art that numerous modifications and variations of the described examples and embodiments are possible in light of the above teaching. The disclosed examples and embodiments may include some or all of the features disclosed herein. Therefore, it is the intent to cover all such modifications and alternate embodiments as may come within the true scope of this invention.

## Claims

1. An injectable aqueous solution comprising a drug formulation not containing nanoparticles and comprising a first, a second and a third component, the first component comprising at least one macrocyclic triene immunosuppressive compound having the following structure: 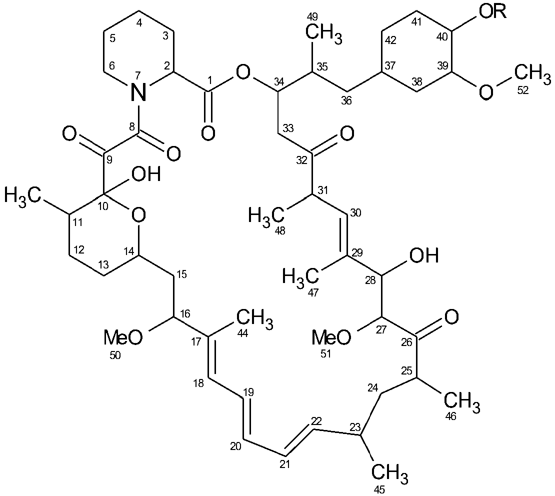 wherein R being C(O)-(CH₂)ₙ-X has one of the following structures: 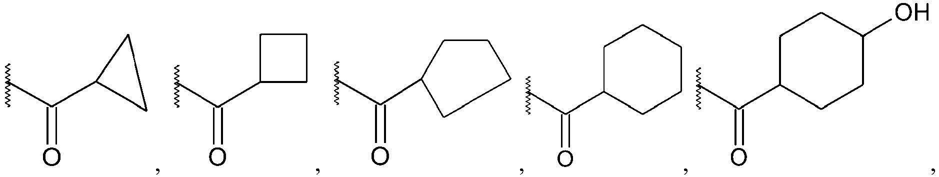 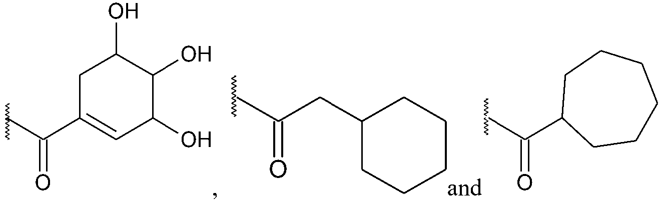 the second component comprising at least one water soluble solubilizer, wherein the first component is solubilized in the second component, and the third component comprising a water soluble polymer, wherein the water soluble polymer is a human serum protein.

2. The injectable aqueous solution according to claim 1, wherein the water soluble polymer is a globular serum protein having an approximate molecular weight of between 65-70 kD.

3. The injectable aqueous solution according to claim 1 or 2, wherein the globular serum protein is a human serum protein having at least 90% homology to SEQ ID NO: 1.

4. The injectable aqueous solution according to any preceding claims, wherein the third component is provided as a solution of the water soluble polymer in physiological saline.

5. A method of manufacturing a drug formulation for parenteral administration, comprising:
(a) providing a first component comprising at least one of a macrocyclic triene immunosuppressive compound having the following structure: 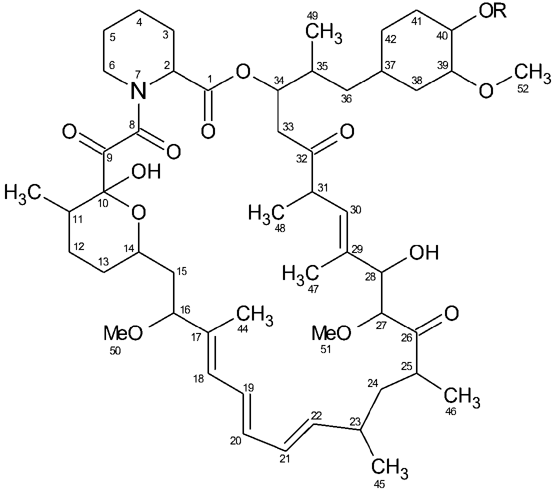 wherein R being C(O)-(CH₂)ₙ-X has one of the following structures: 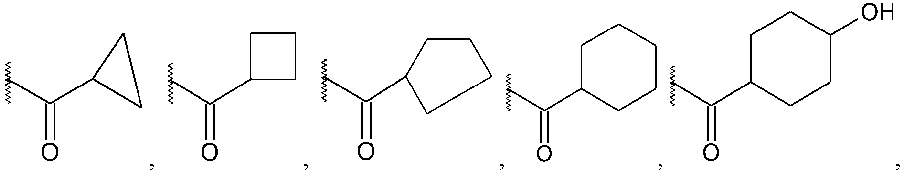 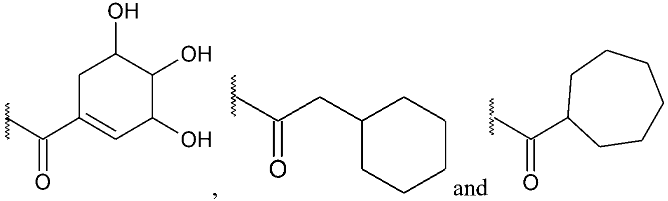
(b) solubilizing the component of (a) in a second component comprising an effective amount of a water soluble solubilizer; and
(c) dispensing the product of (b) in a third component comprising a solution comprising a water soluble polymer , wherein the water soluble polymer is a human serum protein; and
not comprising a particular step of forming nanoparticles.

6. The method of claim 5, wherein the method does not comprise a particular step of forming spherical uniformly sized regular nanoparticles of the macrocyclic triene immunosuppressive compound.

7. A kit containing the first, the second and the third components of any of claims 1 to 4 in pre-weighed and/or premixed combinations thereof and not containing nanoparticles in sterile container(s) to allow ready parenteral administration.
